# EUROPEAN PATENT SPECIFICATION

(11) **EP 4 135 631 B1**
(45) Date of publication and mention of the grant of the patent: **28.10.2026**
(21) Application number: 21916074.4
(22) Date of filing: 30.12.2021
(51) Int. Cl.: A61F 2/60

(54) **A TRANSFEMORAL PROSTHESIS FOR WALKING, SITTING-STANDING, STAIR CLIMBING**
TRANSFEMORALE PROTHESE ZUM GEHEN, SITZEN UND TREPPENSTEIGEN
PROTHÈSE TRANSFÉMORALE PERMETTANT DE MARCHER, D'ADOPTER LA POSITION ASSIS-DEBOUT, DE MONTER DES ESCALIERS

(30) Priority: 30.12.2020 TR 202022508
(43) Date of publication of application: 22.02.2023
(73) Proprietor: Ozyegin Universitesi, 34794 Cekmekoy/Istanbul (TR)
(72) Inventor: UNAL, Ramazan, 34794 Cekmeköy/Istanbul (TR); GEDIKSIZ, Ege, 41050 Izmit/Kocaeli (TR)
(74) Representative: Genç Ilhan, Oznur
(86) International application number: PCT/TR2021/051620
(87) International publication number: WO 2022/146397

(56) References cited:
- EP-A1- 2 913 031
- WO-A1-2012/177125
- WO-A1-2019/228769
- FR-A1- 2 770 772
- US-A1- 2004 181 289
- US-A1- 2005 216 097
- US-A1- 2013 310 949
- FLYNN LOUIS, GEEROMS JOOST, JIMENEZ-FABIAN RENE, HEINS SOPHIE, VANDERBORGHT BRAM, MUNIH MARKO, MOLINO LOVA RAFFAELE, VITIELLO NICO: "The Challenges and Achievements of Experimental Implementation of an Active Transfemoral Prosthesis Based on Biological Quasi-Stiffness: The CYBERLEGs Beta-Prosthesis", FRONTIERS IN NEUROROBOTICS, vol. 12, 1 January 2018 (2018-01-01), XP055958054, DOI: 10.3389/fnbot.2018.00080

## Description

### TECHNICAL FIELD

The invention relates to a transfemoral prosthetic device.

### BACKGROUND ART

The design of transfemoral prosthetic devices is of great interest in current research projects due to the vital impact of availability for amputees. However, in these devices, it is very difficult to provide sufficient mobility by using energy in an efficient manner. In order to preserve metabolic energy and to efficiently simulate the energy in a person's natural gait cycle, energy must be able to be stored, and released, in the ankle and knee. The natural gait cycle involves movement of the thigh, calf, ankle, and foot to allow a person to walk. It is essential for designing a transfemoral prosthesis device to understand the movement of these body parts and their kinematic relations with each other during the natural gait cycle.

Knee prosthesis devices that are currently on the market can be classified as passive, microprocessor-controlled or motorized devices. Due to the fact that those in the passive group are developed in accordance with the kinematics of walking and have fixed characteristics, they are not suitable for changing environmental conditions, walking speed and movements. But they are cheap, lightweight, compact and aesthetically pleasing. Some of these passive prosthetic devices employ specific kinematic configurations to move different parts of the lower limb prosthetic device. Said devices are developed to use some dynamics of walking so as to render them functional. However, individuals with amputations consume approximately 65% more metabolic energy as they cannot fulfill the energy efficiency provided by the muscles and tendons of a biological leg, since there is no energy storage and inter-articular conversion in the existing designs.

Microprocessor-controlled prosthetic devices are used to provide a prosthetic device that simulates natural gait cycle. Such microprocessor-controlled devices are intrinsically passive, using internal actuators to adapt to changing environment and walking speed, and a microprocessor to sense and control these actuators. Thus, human gait is imitated and the compatibility of the controlled prosthetic device is improved compared to the passive prostheses. However, the need for motor and battery increases the weight, and also they are quite expensive compared to passive ones. In addition, the control of these devices also implies some challenges.

Passive and microprocessor-controlled prostheses increase metabolic energy consumption as they do not provide net power to the joint and are not suitable for movements other than walking. In active prosthetic devices used to solve this problem, it is intended to facilitate climbing the stairs by giving net (positive) power to the knee joint of the prosthetic device during the gait cycle. This type of prosthetic device can also reduce the consumption of extra metabolic energy by changing the actual muscle activity to bend and straighten the knees by controlling the motorized parts to simulate the natural gait cycle. However, it is quite large, heavy and very expensive due to the need for a large motor and battery.

Since these passive, microprocessor-controlled and motorized knee prosthesis devices available on the market do not have a connection with the foot joint, they do not offer energy conversion, which is very important in walking and other movements. For this reason, they cannot provide natural leg functions and moreover, the external energy required in cases where the amputee's own metabolic energy is not sufficient.

Recently, the design of transfemoral prosthetic devices incorporating such movements has been attempted to be improved using energy storage components in order to reduce power consumption of the transfemoral prosthetic device during a gait cycle, however, such improvements have failed to maximize the total energy generated from the natural gait cycle.

"The Challenges and Achievements of Experimental Implementation of an Active Transfemoral Prosthesis Based on Biological Quasi-Stiffness: The CYBERLEGs Beta-Prosthesis" describes an active transfemoral prosthesis to create an average ground gait in both the knee and ankle in the sagittal plane. However, there is a need to provide more efficient locking mechanisms to make better use of this prosthesis. In addition, it is stated that the users deviate from the natural gait cycle while using the prosthesis.

In the patent document No. US2013310949 describes an apparatus and method for controlling climbing and descending stairs for electrical lower limb devices. The system processes the data received from a real-time sensor and controls only the knee joint and foot joint of the prosthesis. However, these two joints are not sufficient to simulate the natural gait cycle.

In the patent document No. WO213157965 describes a prosthesis that drives the knee joint and ankle joint by means of an electric motor. However, the prosthesis is quite large and heavy with its motor and gear system.

US 2004/181289 A1 is directed to a prosthesis device comprising a knee member, an elongated trans-tibial member having opposite top and bottom ends spaced apart along a main longitudinal axis, and an actuator comprising a motor, wherein the motor induces a corresponding rotation of said knee joint relative to the lower leg about a pivot axis. US 2004/181289 A1 is mainly focused on pivot assemblies, wherein said motor is pivotally connected to said trans-tibial member via a second pivot assembly defining a second pivot axis and said lower leg is pivotally connected to said knee joint via a third pivot assembly at a location spaced from said first pivot assembly and thereby defining a third pivot axis. Document US 2004/181289 A1 is considered to represent the prior art closest to the device defined by independent claim 1.

US 2005/216097 A1 discloses an artificial prosthetic or orthotic foot for a human including an artificial midfoot joint, an artificial metatarsophalangeal (MTP) joint, and a mechanical coupler configured to coordinate movement of the artificial midfoot joint relative to the artificial MTP joint. These two joints are created using "tensegrity" design principles, where connections between the compression members are made by a network of tension members which create axes of motion, and limitations on those axes of motion. US 2005/216097 A1 aims to coordinate movement of the artificial midfoot joint relative to the artificial MTP joint and to duplicate the rollover profile of the human foot.

WO 2019/228769 A1 relates to a thigh prosthetic component has a knee joint, a foot part unit with an ankle as well as a lower leg unit, and aims to provide an energy efficient and stable natural gait pattern for the patient.

The invention relates to an upper leg prosthesis adapter for connecting to an upper leg shaft, having a knee joint which can be pivoted between an extended position and a flexed position, comprising a proximal knee joint upper part and a distal knee joint upper part which are connected together via a ventral joint arm and a dorsal joint arm, wherein the ventral joint arm is connected to the proximal knee joint upper part in an articulated manner via a ventral upper knee joint axis and to the distal knee joint lower part in an articulated manner via a ventral lower knee joint axis. The dorsal joint arm is connected to the proximal knee joint upper part in an articulated manner via a dorsal upper knee joint axis and to the distal knee joint lower part in an articulated manner via a dorsal lower knee joint axis, and a foot joint for pivotally connecting a foot part to a lower leg unit connected to the knee joint. The aim of the invention is to provide an upper leg prosthesis adapter which allows a natural gait and which is more energy-efficient for the patient and better prevents against falling

FR 2 770 772 A1 discloses a leg prosthesis including a femoral part, a tibial part and a foot articulated together. The femoral and tibial parts are articulated together to form a knee joint and the tibial and foot parts are articulated together to form an ankle joint. A piston which is movably mounted in a hydraulic damping and locking transfer chamber to oppose the flexion of the knee joint when the foot part is under load and actuates means for controlling a hydraulic transfer closing member. The piston aims of dampening movement of the knee.

Given the designs in the prior art, there is still a need for an improved prosthetic device that imitates the energetic behavior that occurs during a human gait cycle, having the functionality of an active or microprocessor-controlled prosthetic device and the simplicity of a passive transfemoral prosthetic device, maximizing the absorption, storage and release of the energy produced during different movements such as sitting-standing, stair climbing-descending and walking, which naturally adapts to different speeds, slopes and surfaces, and ensuring the necessary energy efficiency.

### SUMMARY OF THE INVENTION

Particular embodiments of the invention include a smart/microprocessor-controlled prosthesis that provides natural speed and metabolic energy consumption in walking, by means of a lightweight and compact product that acts like a passive prosthesis and is controlled by mini motors, and an active prosthesis that fully performs functions (such as stair climbing) of the biological leg.

The invention is defined by independent claim 1. Advantageous embodiments are defined by the dependent claims. Thus, the device according to the invention includes a foot part, a knee joint, and an ankle joint, wherein the upper leg of the user is connected to the lower leg through the knee joint, wherein the lower leg is connected to the foot part through the ankle joint. This transfemoral prosthesis can store and release energy in both joints, namely the ankle and knee. The knee joint stores energy during the swing phase and transfers it through a connector to the ankle joint during the push-off. The swing storage spring-gear mechanism moves this element during the push-off (ankle push-off, the phase between heel lift-off and toes lift-off) to release the stored energy in a natural manner. It uses a balancing spring to prevent the foot from hitting the ground again after the push is terminated. This provides safe ground clearance during swing and brings the foot to the starting position (an ankle joint of 0 degree) before the heel strike. After the heel strike, a weight-receiving spring in the knee joint stores and releases energy in the same joint to ensure safe descending and efficient ankle roll-over. This spring is disengaged during swing and activated by the heel strike. The same spring stores energy during sitting to provide energy-saving sit-to-stands and provides sit-to-stand support by returning it back when standing up. The same spring is also used to store energy in parallel with a motor during stair climbing in order to provide an energy efficient and lightweight solution to robotic prostheses. A moment arm of the weight-receiving spring is modified to increase and decrease the torque around the knee joint in accordance with different walking speeds.

The numerous advantages, features, and functions of the various embodiments herein will be readily apparent and better understood in the light of the following description and accompanying drawings. The following description is not intended to limit the scope of the transfemoral prosthesis assembly, but rather provides exemplary embodiments for ease of understanding.

Hereinafter, the present invention will be mainly described with reference to a prosthetic device. It should be understood, however, that the invention equally applies to an orthotic device with similar effects. An example of a device according to the present invention is a transfemoral prosthetic device that simulates an energy-efficient absorption and release of a natural knee joint and ankle joint during the gait cycle.

### BRIEF DESCRIPTION OF THE DRAWINGS

An embodiment of the present invention is illustrated by way of example in the accompanying drawings for better understanding thereof, and their use will be more readily apparent in view of the detailed description where reference numerals denote same or similar items, and the following drawings, wherein:

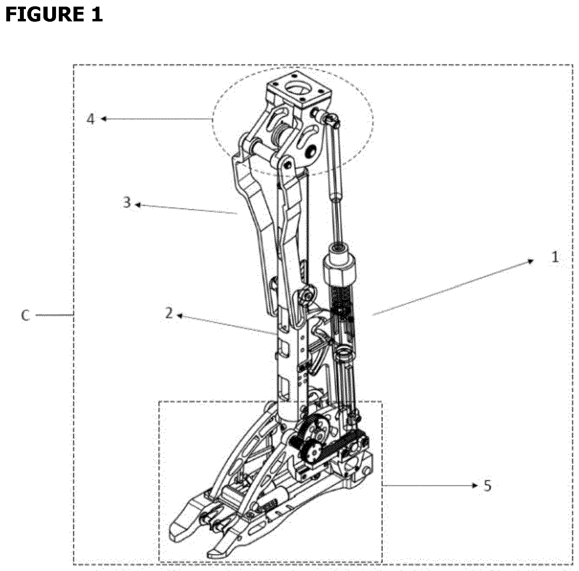

Figure 1 is a view of a prosthesis in an embodiment of the present invention.

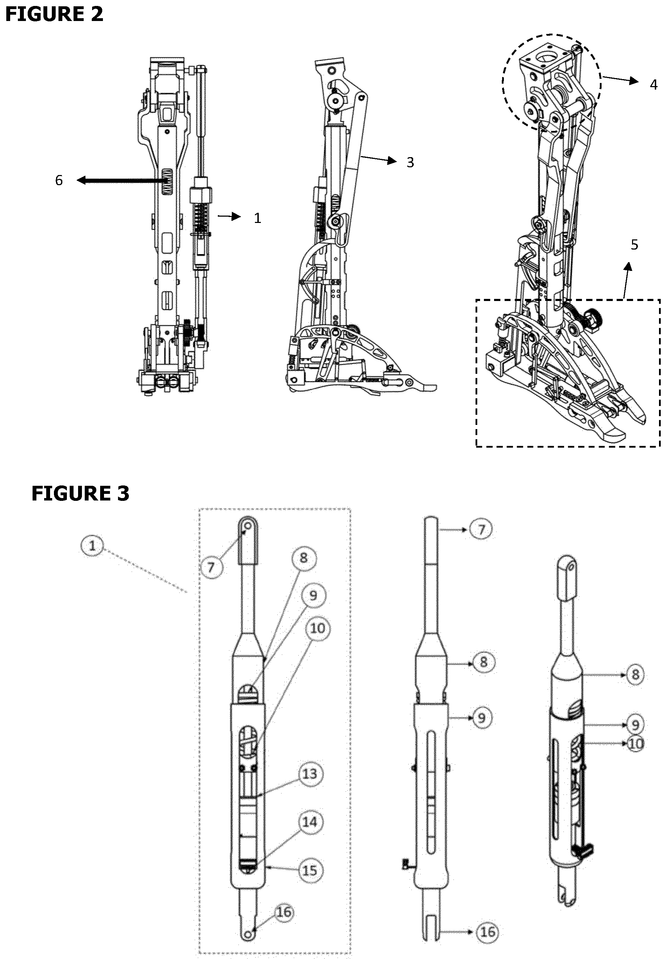

Figure 2 includes views of the prosthesis from different angles, in an embodiment of the present invention.
Figure 3 includes views of a swing storage mechanism from different angles, in an embodiment of the present invention.
Figure 4 includes views of a knee joint of the prosthesis from different angles, in an embodiment of the present invention.
Figure 5 includes views of a weight-receiving mechanism of the prosthesis from different angles, in an embodiment of the present invention.

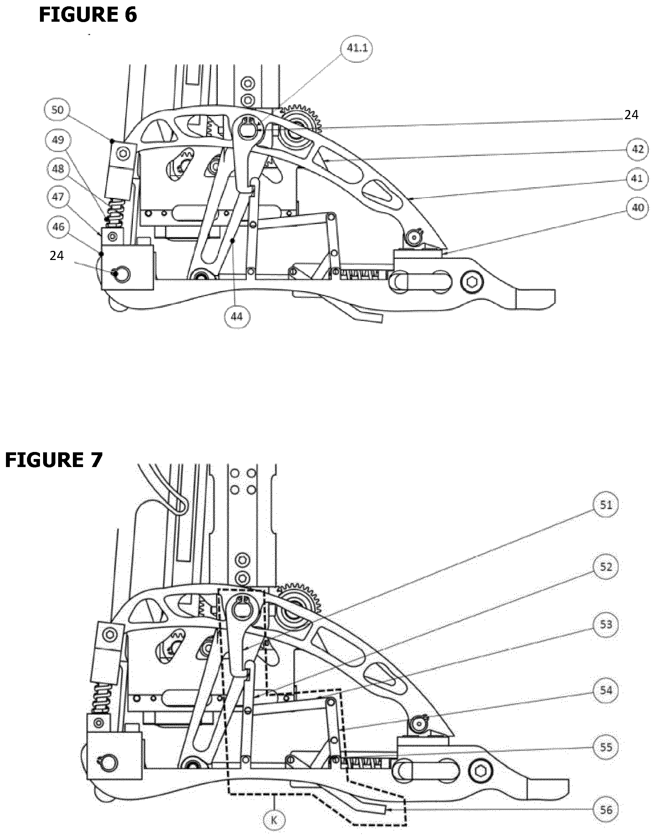

Figure 6 is a side view of a foot part of the prosthesis in an embodiment of the present invention.
Figure 7 is a view of an ankle lock mechanism of the prosthesis in an embodiment of the present invention.

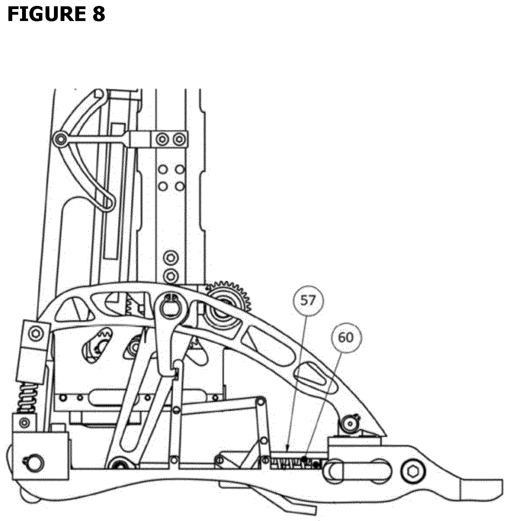

Figure 8 is a view of a dorsiflexion energy storage mechanism provided in the foot part of the prosthesis in an embodiment of the present invention.

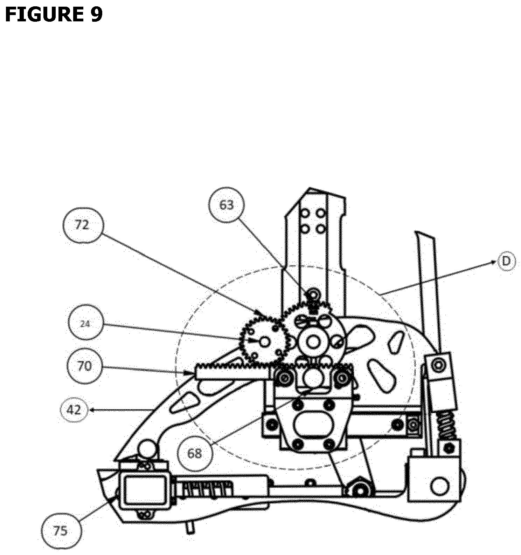

Figure 9 is a view of a swing storage spring-gear mechanism of the prosthesis in an embodiment of the present invention.

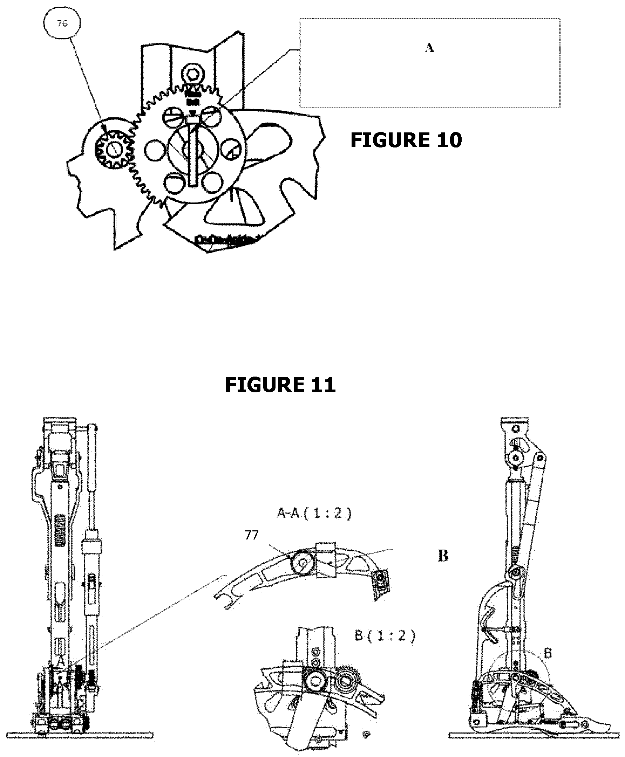

Figure 10 is a view of two of the gears of the swing storage spring-gear mechanism of the prosthesis in an embodiment of the present invention.
Figure 11 is a view of a balancing spring provided in the foot part of the prosthesis in an embodiment of the present invention.

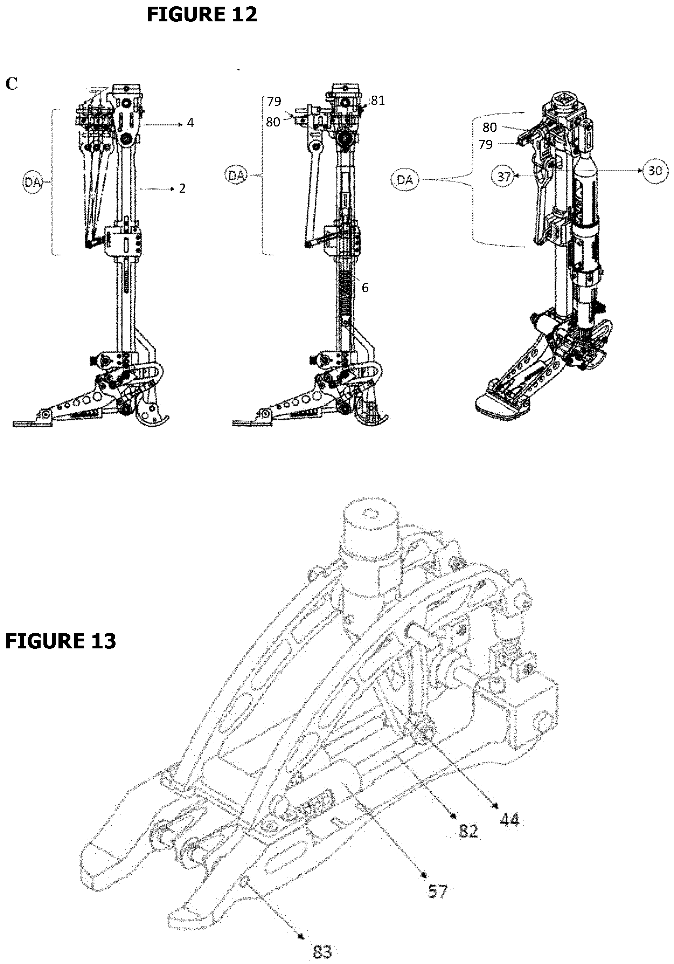

Figure 12 is a view of a posture adaptation mechanism in an embodiment of the present invention.
Figure 13 is a view of the dorsiflexion energy storage mechanism, from another angle, in an embodiment of the present invention.

The elements shown in the figures are denoted the following numbers:
1. Swing storage mechanism
2. Lower leg
   2.1 Lower leg housings
3. Weight-receiving mechanism
4. Knee joint
5. Foot part
6. Weight-receiving member
7. housing of the upper tube
8. Upper tube
9. Spring cover
10. Swing storage spring
13. Guide screw
14. BLDC motor
15. Lower tube
16. housing of the lower tube
17. Left flange
   17.1 First housing of the knee joint
   17.2 Second housing of the knee joint
   17.3 Third housing of the knee joint
19. Potentiometer
22. Right flange
24. Shaft
26. Upper part of the knee
   26.1 Upper housings of the knee
30. Left compression arm
   30.1 Compression spring housing
   30.2 Housing of the weight-receiving spring
33. Support arm
34. Lock fork
   34.1 Lock fork housing
37. Right compression arm
40. Foot middle part
41. Right foot side
   41.1 Ankle member housing
42. Left foot side
44. Ankle member
46. Right heel spring holder
47. Left heel spring holder
48. Heel spring rod
49. Heel springs
50. Heel spring connector
51. Ankle shaft gear
52. First member of the ankle lock
53. Second member of the ankle lock
54. Third member of the ankle lock
55. Fourth member of the ankle lock
56. Fifth member of the ankle lock
57. Dorsiflexion spring tube
60. Dorsiflexion spring
63. Drive gear
68. Gear rod pin
70. Gear rod
72. Driven gear
75. Inertial sensor
76. Intermediate gear
77. Balancing spring
79. Guide rail
80. Carrier
81. Motor
82. Piston rod
83. Toe tip housings
C. Prosthetic or orthotic device
D. Swing storage spring-gear mechanism
DA. Posture adaptation mechanism
K. Ankle lock mechanism

### DETAILED DESCRIPTION OF THE INVENTION

An orthotic or prosthetic device is developed by imitating natural movements and energetic behavior of different body parts by analyzing the energetic and kinematic relationships that occur during different movements.

An ankle and knee have different energy absorption ranges during a natural human gait cycle. These energy absorption ranges around the ankle and knee can be used to imitate a natural gait cycle, optimizing the generation of energy required for a push-off.

A natural gait cycle consists of stance, front-swing, and swing phases, during which the knee joint (4) is mainly an energy absorber while the ankle joint is mainly an energy generator. In the stance phase, that is, between about 0% and about 45% of a stride, the knee absorbs energy during knee flexion and generates approximately same amount of energy for knee extension. Meanwhile, the ankle joint absorbs energy during dorsiflexion of the ankle, which is generated between about 10% and 45% of the stride. During the pre-swing phase, the knee absorbs about 50% to about 70% of the gait cycle. On the other hand, the ankle generates the majority of the energy required for push-off (approximately 80% of the total power generation during the entire gait cycle) between about 45% and 60% of the gait cycle. In the swing phase, the knee absorbs energy between about 80% and 95% of the gait cycle, while the power flow around the ankle is negligible. As a result, a natural gait power flow can be provided to a transfemoral prosthetic device based on the relationship of the absorption and energy generation around a person's knee and ankle joint.

The transfemoral prosthetic or orthotic device (C), formed using these kinetic relationships, comprises a swing storage mechanism (1), a lower leg (2), a weight-receiving mechanism (3), a knee joint (4), and a foot part (5) to imitate different energy absorption and generation intervals occurring around the knee and ankle joints during a natural gait cycle.

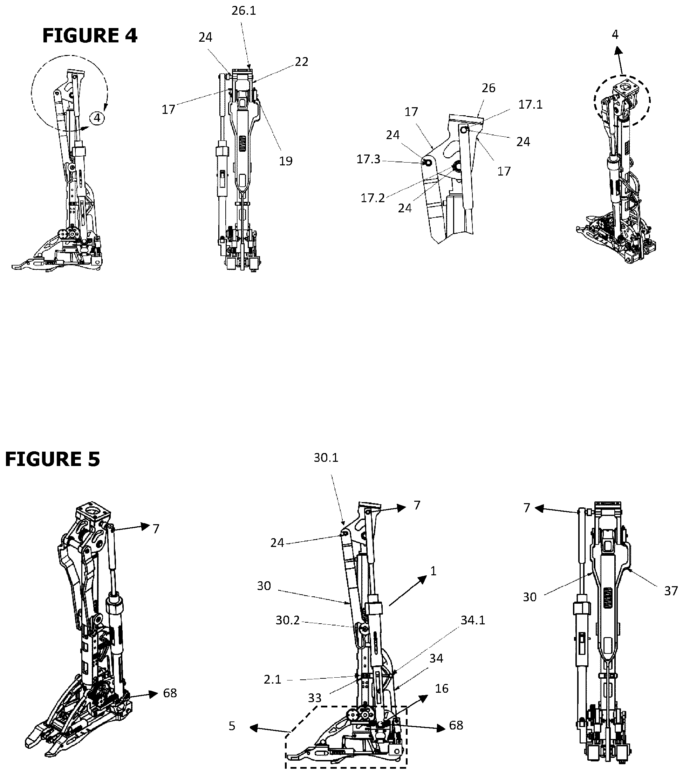

The swing storage mechanism (1) connects the knee joint (4) to the foot part (5), simulating the energy absorption of the knee joint (4). It is responsible for the absorption of energy in the swing phase and its transfer to the foot part (5). Thus, it is made possible to use the energy stored in the knee, on the ankle. In an embodiment of the device, the swing storage mechanism (1) consists of a nested upper tube (8) and a lower tube (15). As can be seen in Fig. 5, the swing storage mechanism (1) is connected to the foot part (5) at the housing of the lower tube (16) by means of the gear rod pin (68). As for the knee joint (4), it is connected to the first housing of the knee joint (17.1) at the housing of the upper tube (7) by means of a shaft (24). A spring cover (9) located in the nested upper tube (8) and the lower tube (15) moves with the guide screw (13) to change an activation position of the connection member swing storage spring (10).

The swing storage mechanism (1) also serves for the partial absorption of the energy in the ankle roll-over part of the stance phase of the gait cycle and for the transfer of this energy to the ankle part for push-off. The swing storage mechanism (1) may comprises another mechanical device or configuration capable of absorbing and releasing energy. Furthermore, the swing storage mechanism (1) may be attached to the knee joint (4) by fastening mechanisms known in the art, such as pins, screws, bolts or other mechanical means fixing the positioning of. The housing of the lower tube (16) at the other end of the swing storage mechanism (1) is attached to a movable device such as a slider, bolt, pin, carriage, roller, or other slideable device. Thus, the swing storage mechanism (1) can be transported along a guided path in the foot part (5) between at least two positions. One skilled in the art will appreciate that said connection points (7, 16) can be reversed or repositioned.

In an embodiment of the device, the spring cover (9), the guide screw (13) and the BLDC motor (14) connection member form the adaptation mechanism within the swing storage mechanism (1). This mechanism changes the position of the swing storage spring (10) and activates the swing storage spring (10) at different times in accordance with different walking speeds and adjusts the swing angle of the knee. In this way, the position of the swing storage spring (10), and therefore the stored energy, is adapted to store the energy generated at different walking speeds. The swing adaptation mechanism may be used in another mechanical device or configuration that can change its position, such as a hydraulic or pneumatic cylinder, instead of the swing storage spring (10).

The lower leg (2) is connected to the ankle member (44) of the foot part (5) through the ankle member housing (41.1) by means of a shaft (24) located on the foot part (5). In an embodiment of the device, a weight-receiving member (6) is provided inside the lower leg (2) that ensures safe descending and effective ankle roll-over in the stance phase. The lower leg (2) may be made of an aluminum alloy, steel, carbon fiber, plastic or similar material having suitable strength and physical properties. In a basic embodiment of the invention, a spring is used as a weight-receiving member (6). Furthermore, in other embodiments of the invention, another mechanical device or configuration capable of absorbing and releasing energy may be used as the weight-receiving member (6).

The weight-receiving mechanism (3) is used to enable safe descending and effective ankle roll-over during the stance phase. In an embodiment of the device, the left compression arm (30) and the right compression arm (37) are configured (hereinafter referred to as the compression arm (30, 37)) for the weight-receiving mechanism (3) to compress the weight-receiving member (6). The compression arm (30, 37) is connected to the knee joint (4) via the compression spring housing (30.1) and the third housing of the knee joint (17.3). This connection is performed by means of a shaft (24). The weight-receiving mechanism (3) is also connected to the weight-receiving member (6) through the housing of the weight-receiving spring (30.2). A lock fork (34) is provided herein, which is configured for activating and deactivating the weight-receiving mechanism (3). The lock fork (34) is connected to the lower leg (2) through the lock fork housing (34.1) via the lower leg housings (2.1) and the support arm (33). The weight-receiving mechanism (3), which is connected to the weight-receiving member (6) through the compression spring housing (30.1), is released when the foot is lifted off the ground and the compression arm (30, 37) does not press the weight-receiving member (6), thereby releasing the knee in the swing phase. Once the foot touches the ground, the compression arm (30, 37) sits on the weight-receiving member (6) and presses weight-receiving member (6). With the sit-to-stand support mechanism, the weight-receiving mechanism (3), which is configured to allow flexion of the knee during sitting, presses the weight-receiving member (6) to store energy, and returns it back while standing up, thereby providing sitting and standing support.

In another embodiment of the invention, with the sit-to-stand support mechanism, the weight-receiving mechanism (3), which is configured to allow 75°-170° flexion of the knee, more specifically 110° flexion while sitting, presses the weight-receiving member (6) and stores energy, and returns it back while standing up, thereby providing sitting and standing support.

In the present invention, the compression arm (30, 37) and the lock fork (34) may be made of an aluminum alloy, steel, carbon fiber, plastic, or similar material with appropriate strength and physical properties.

In another embodiment of the invention, another mechanical device or configuration capable of compressing and releasing the weight-receiving member (6) can be used instead of the weight-receiving mechanism (3).

The compression arm (30, 37) can be attached to the knee joint (4) with pins, screws, bolts, or it can be attached to the knee joint (4) by fastening mechanisms known in the art such as other mechanical means fixing the positioning of the compression arm (30, 37) through the compression spring housing (30.1). The lower end of the compression arm (30, 37) fully fits into the weight-receiving spring housing (30.2) and compresses the weight-receiving member (6), and comes out off the weight-receiving spring housing (30.2), thereby releasing the weight-receiving member (6).

According to the present invention, the lock fork (34) can be attached to the lower leg (2) by fastening mechanisms known in the art, such as pins, screws, bolts, or other mechanical means that fix the lock fork (34) to the lower leg (2) through the lower leg housings (2.1). Instead of the lock fork (34), another mechanical device or configuration capable of locking or releasing the compression arm (30, 37) may be used.

The user's upper leg is connected to the lower leg (2) of the device by means of the knee joint (4). In an embodiment of the device, the upper leg of the user is connected through the upper housings (26.1) of the device on the upper part (26) of the knee joint (4). The lower leg (2) is connected to the second housing (17.2) of the knee joint (4) by means of a shaft (24). The lower leg (2) is also pivotally connected to the knee joint (4) by means of a shaft (24). The left flange (17) and the right flange (22) secure the compression arm (30, 37) of the weight-receiving mechanism (3) to the third housing of the knee joint (17.3) by means of a shaft (24). The swing storage mechanism (1) is connected to the knee joint (4) via the housing of the upper tube (7) and the first housing of the knee joint (17.1). It includes a potentiometer (19) to measure the angle of the knee joint (4).

In another embodiment of the invention, another mechanical device or configuration capable of connecting or releasing the user's upper leg to the lower leg (2) may be used.

In another embodiment of the invention, a posture adaptation mechanism (DA) is configured to increase or decrease the torque formed around the knee in accordance with the walking speed by changing the position of the compression arm (30, 37) during weight-receiving. The compression arm is adjusted in the knee axis by the movement of a carrier (80) on the guide rail (79) in accordance with the walking speed. The walking speed is determined by measuring the angle and angular velocity of the knee by a potentiometer (19), and accordingly, the motor (81) moves the carrier (80) on the guide rail (79) in order to provide the required torque around the knee. Thus, the pressing force of the compression arm on the weight-receiving member (6) can be changed.

The foot part (5) of the device according to the present invention comprises a foot middle part (40) forming the skeleton of the foot part (5), a right foot side (41), a left foot side (42), a balancing spring (77) inserted in the ankle member housing (41.1) located in the ankle member (44), a swing storage spring-gear mechanism (D), an ankle lock mechanism (K), and heel springs (49). Here, the heel springs (49) are used to cushion the ground strike. The right foot side (41) and left foot side (42) are arranged in a hollow structure. This structure ensures that the prosthesis is greatly lightened and has an ergonomic structure. The ankle lock mechanism (K) is formed by an ankle shaft gear (51), a first member of the ankle lock (52), a second member of the ankle lock (53), a third member of the ankle lock (54), a fourth member of the ankle lock (55), and a fifth member the ankle lock (56). When the fifth member of the ankle lock (56) touches the ground, the ankle shaft gear (51) and the first member of the ankle lock (52) are engaged and the ankle joint is locked.

The dorsiflexion spring tube (57) containing the dorsiflexion spring (60) is connected to the front of the foot part (5) through the toe tip housings (83), and the piston rod (82) pressing the dorsiflexion spring tube (57) is connected to the ankle member (44) at the back of the foot. The dorsiflexion spring (60) stores energy during dorsiflexion of the ankle, which is generated about 10% and 45% of the stride.

The balancing spring (77) according to the present invention is configured to allow the foot to return to its normal position after the push-off. In an embodiment of the invention, it is positioned between the balancing spring (77) and the front of the foot and the ankle member (44). It may include another mechanical device or configuration that can be replaced with a structure that will allow the foot to return/reset to its normal position after the push-off.

In an embodiment of the device, the mechanism moving the swing storage spring (10), which is configured such that a near-natural energy generation can be achieved during push-off, is formed by the drive gear (63), the driven gear (72), the intermediate gear (76) and the gear rod (70). The drive gear (63) is configured to rotate with respect to the movement of the ankle. The drive gear (63) is coupled onto the left foot side (42). The intermediate gear (76) is configured to transfer the motion of the drive gear (63) to the driven gear (72). It is connected between the foot left side (42) and the driven gear (72) by means of a shaft (24). The driven gear (72) is configured to allow the gear rod (70) and the swing storage mechanism (1) attached thereto to move back and forth. The mechanism that moves the swing storage spring (10) may also be another mechanical device or configuration that can be replaced by a movable device such as a slider, bolt, pin, carriage, roller, or other sliding device. Thus, the swing storage mechanism (1) can be moved between at least two positions in the foot part (5) by being moved along a guided path.

The heel springs (49) provide shock absorption. In an embodiment of the invention, the heel springs (49) and the heel spring rod (48) are fixed to the foot part (5) at the lower end by means of the right heel spring holder (46) and the left heel spring holder (47). The movement of the heel springs (49) is limited by a shaft (24). The heel springs (49) are fixed to the foot right and left sides (41, 42) at the upper end by means of the heel spring connector (50). Also, another mechanical device or configuration that provides shock absorption can be used.

This transfemoral prosthesis can store and release energy in both joints, namely the ankle and knee. The knee joint (4) stores energy during swing and transfers it to the ankle joint during pushing through the swing storage mechanism (1). The actuation mechanism for the swing storage spring (10) moves the swing storage mechanism (1) during pushing to naturally release the stored energy. After the push-off, the balancing spring (77) retracts the foot so that the ankle joint is 0 degrees. This provides safe ground clearance during swing and resets the foot to the starting position before the heel strikes. After the heel strike, the weight-receiving member (6) at the knee joint (4) stores and release energy at the same joint to ensure safe descending and efficient ankle roll-over. This weight-receiving member (6) is deactivated during swing and activated by the heel strike. The same spring stores energy during sitting and returns it back while standing up in order to provide energy-efficient sit-to-stands. The same spring is also used to store energy in parallel with a motor (81) during stair climbing in order to provide an energy efficient and lightweight solution to robotic prostheses.

The energy storage capacity of the swing storage mechanism (1) is increased and decreased in accordance with the ankle push-off variance, and in order to adapt to the knee angular velocity of the swing phase for different walking speeds. The moment arm of the weight-receiving spring is modified to increase and decrease the torque around the knee joint (4) in accordance with the different walking speeds. In an embodiment of the invention, the inertial sensor (75) is configured in the front part of the foot to measure the angular velocity of the foot in controlling the adaptive and robotic transfemoral prosthesis.

The weight-receiving member (6) and the swing storage mechanism (1) located in the lower leg (2) of the prosthesis are provided with motor (81) and microprocessor control, thus providing an active device that imitates the movement and energetic behavior of the human natural knee and ankle joints, which is simplified and non-bulky, and which stores and release energy during different phases of the natural gait cycle.

While described and illustrated by the above embodiments, it is understood that alternatives and modifications to these embodiments, such as those suggested by others, may be included in the scope of the specification. Furthermore, any of the principles described herein can be extended to other orthopedic devices, prosthetic devices, which require similar functions of the structural members described herein. For example, the use of the above elements is not limited to a transfemoral prosthetic device, but may also be used in a transtibial prosthetic device or similar lower leg (2) prosthetic device.

The present invention is a passive prosthesis that combines the knee and ankle with energy conversion suitable for the nature of walking, which provides a passive prosthesis device that reduces the excessive metabolic energy consumption and increases the normal walking speed of the user, by being optimized for walking, which constitutes 90% of the leg functions in daily life, and allowing other functions to be performed. It is also possible to provide an active device that stores and releases energy at different phases of the natural gait cycle by providing motor (81) and microprocessor control to the prosthesis, which is simplified and not bulky, and which imitates the movement and energetic behavior of the human natural knee and ankle joints.

### MEANINGS OF THE SIGNS IN THE FIGURES

### FIGURE 10

**A=** Groove for a motion range of 25 degrees for the bolt in the drive gear. Not for rotating the drive gear during dorsiflexion.

### FIGURE 11

**B=** The surface determines the activation of the resetting spring based on the position of the ankle.

### FIGURE 12

**C=** The moment arm is adjusted to the knee axis by screw-motor activation.

## Claims

1. A prosthesis device (C) comprising:
- a knee joint (4) connectable with the user's leg;
- a lower leg (2) connecting the knee joint (4) and a foot part (5) to each other, wherein the lower leg (2) is connected to the knee joint (4), and the lower leg (2) is connected to the foot part (5) through an ankle member housing (41.1);
- a swing storage mechanism (1) connected to the foot part (5) by means of a housing of the lower tube (16) and a gear rod pin (68), and to the knee joint (4) by means of a housing of the upper tube (7) and a first housing of the knee joint (17.1),
- the foot part (5) comprises a dorsiflexion spring (60) and a swing storage spring-gear mechanism (D) wherein the swing storage spring-gear mechanism (D) comprising a balancing spring (77) inserted in the ankle member housing (41.1) located in an ankle member (44) wherein the balancing spring (77) is configured to allow the foot, and also ankle, to return to its normal position after push-off, the swing storage spring-gear mechanism (D) further comprises a drive gear (63), a driven gear (72), an intermediate gear (76) and a gear rod (70) wherein the drive gear (63) is configured to rotate with respect to movement of the ankle, the intermediate gear (76) is configured to transfer the motion of the drive gear (63) to the driven gear (72) which is configured to allow the gear rod (70) and the swing storage mechanism (1) attached to said gear rod (70) to move back and forth, to enable the swing storage mechanism (1) to be transported between at least two positions in the foot part (5) by means of the swing storage spring-gear mechanism (D);
wherein the swing storage mechanism (1) comprises a swing adaptation mechanism consisted of nested connection member tubes (8, 15) and a connection member swing storage spring (10) located in the tubes, a spring cover (9), a guide screw (13) and a BLDC motor (14), and the swing adaptation mechanism (1) is configured to change the position of the swing storage spring (10) and to activate the swing storage spring (10) at different times in accordance with different walking speeds and adjusts the swing angle of the knee,
wherein a dorsiflexion spring tube (57) containing the dorsiflexion spring (60) is connected to the front of the foot part (5) through a toe tip housings (83), and a piston rod (82) pressing the dorsiflexion spring tube (57) is connected to the ankle member (44) at the back of the foot,
the prosthesis device (C) also comprising:
- a weight-receiving mechanism (3) comprising a weight-receiving member (6) located in the lower leg (2), a compression arm (30, 37) and a lock fork (34) for activating and deactivating the weight-receiving member (6),
wherein the compression arm (30, 37) is connected to the knee joint (4) through a compression spring housing (30.1) and a knee joint third housing (17.3) and a shaft (24), and to the weight-receiving member (6) through a weight-receiving housing (30.2),
wherein a lower end of a support arm (33) is connected to the lower leg (2) through lower leg housings (2.1) and other end of the support arm (33) is connected to the lock fork (34) through a lock fork housing (34.1).

2. A prosthesis device (C) according to claim 1, wherein the weight-receiving member (6) is a spring.

3. A prosthesis device (C) according to claim 1 or 2, further comprising a posture adaptation mechanism (DA) comprising a guide rail (79) and a carrier (80) connected to the compression arm (30, 37), wherein the posture adaptation mechanism (DA) adjusts the compression arm (30,37) in the knee axis by the movement of the carrier (80) on the guide rail (79) in accordance with the walking speed determined by the potentiometer (19) by measuring the angle and angular velocity of the knee, in order to increase or decrease the torque formed around the knee in accordance with the walking speed.

4. A prosthesis device (C) according to any one of the preceding claims, further comprising an ankle lock mechanism (K) comprising a first member of the ankle lock (52), a second member of the ankle lock (53), a third member of the ankle lock (54), a fourth member of the ankle lock (55), and a fifth member of the ankle lock (56), the ankle lock mechanism (K) further comprises an ankle shaft gear (51) configured to lock ankle joint by engaging with the first member of the ankle lock (52) in case the fifth member of the ankle lock (56) touches the ground.

5. A prosthesis device (C) according to any one of the preceding claims, wherein the weight-receiving mechanism (3), which is configured to allow 75°-110° flexion of the knee while sitting, is configured to press the weight-receiving member (6) via the compression arm (30, 37) connected to the knee joint (4) and the weight-receiving member (6) and to store energy on the weight-receiving member (6).

6. A prosthesis device (C) according to any one of the preceding claims, wherein heel springs (49) configured to provide shock absorption are connected to the foot part (5) at the lower end by means of a right heel spring holder (46) and a left heel spring holder (47), and are connected to foot right and left sides (41, 42) at the upper end by means of a heel spring connector (50).

7. A prosthesis device (C) according to any one of the preceding claims, further comprising a motor (81) located at an end of the weight-receiving member (6) and providing power by pressing said weight-receiving member (6).

8. A prosthesis device (C) according to any one of the preceding claims, further comprising a control unit adapted to control the motors (81) contained in the prosthetic or orthotic device (C).

## Patentansprüche

1. Eine Prothesenvorrichtung (C), umfassend:
- ein Kniegelenk (4), das mit dem Bein des Anwenders verbindbar ist;
- einen Unterschenkel (2), der das Kniegelenk (4) und ein Fußteil (5) miteinander verbindet,
wobei der Unterschenkel (2) mit dem Kniegelenk (4) verbunden ist und der Unterschenkel (2) über ein Knöchelelementgehäuse (41.1) mit dem Fußteil (5) verbunden ist;
- einen Schwungspeicherungsmechanismus (1), der mittels eines Gehäuses des unteren Rohrs (16) und eines Zahnstangenstifts (68) mit dem Fußteil (5) und mittels eines Gehäuses des oberen Rohrs (7) und eines ersten Gehäuses des Kniegelenks (17.1) mit dem Kniegelenk (4) verbunden ist,
- das Fußteil (5) eine Dorsalextensionsteder (60) und einen Schwungspeicherungs-Feder-Getriebe-Mechanismus (D) umfasst, wobei der Schwungspeicherungs-Feder-Getriebe-Mechanismus (D) eine Ausgleichsfeder (77) umfasst, die in das in einem Knöchelelement (44) angeordnete Knöchelelementgehäuse (41.1) eingesetzt ist, wobei die Ausgleichsfeder (77) so konfiguriert ist, dass sie es dem Fuß und auch dem Knöchel ermöglicht, nach dem Abstoßen in ihre Normalposition zurückzukehren, wobei der Schwungspeicherungs-Feder-Getriebe-Mechanismus (D) ferner ein Antriebszahnrad (63), ein angetriebenes Zahnrad (72), ein Zwischenzahnrad (76) und eine Zahnstange (70) umfasst, wobei das Antriebszahnrad (63) so konfiguriert ist, dass es sich in Bezug auf die Bewegung des Knöchels dreht, das Zwischenzahnrad (76) so konfiguriert ist, dass es die Bewegung des Antriebszahnrads (63) auf das angetriebene Zahnrad (72) überträgt, welches so konfiguriert ist, dass es der Zahnstange (70) und dem an der besagten Zahnstange (70) angebrachten Schwungspeicherungsmechanismus (1) ermöglicht, sich hin und her zu bewegen, um zu ermöglichen, dass der Schwungspeicherungsmechanismus (1) mittels des Schwungspeicherungs-Feder-Getriebe-Mechanismus (D) zwischen mindestens zwei Positionen im Fußteil (5) transportiert wird;
wobei der Schwungspeicherungsmechanismus (1) einen Schwunganpassungsmechanismus umfasst, der aus ineinandergesteckten Verbindungselementrohren (8, 15) und einer in den Rohren angeordneten Verbindungselement-Schwungspeicherungsfeder (10), einer Federabdeckung (9), einer Führungsschraube (13) und einem BLDC-Motor (14) besteht, und der Schwunganpassungsmechanismus (1) so konfiguriert ist, dass er die Position der Schwungspeicherungsfeder (10) verändert und die Schwungspeicherungsfeder (10) in Übereinstimmung mit unterschiedlichen Gehgeschwindigkeiten zu unterschiedlichen Zeiten aktiviert und den Schwungwinkel des Knies einstellt,
wobei ein Dorsalextensionfederrohr (57), das die Dorsalextensionfeder (60) enthält, über Zehenspitzengehäuse (83) mit der Vorderseite des Fußteils (5) verbunden ist, und eine Kolbenstange (82), die auf das Dorsalextensionfederrohr (57) drückt, mit dem Knöchelelement (44) an der Rückseite des Fußes verbunden ist,
die Prothesenvorrichtung (C) ferner umfassend:
- einen Gewichtsaufnahmemechanismus (3), der ein Gewichtsaufnahmeelement (6), das sich im Unterschenkel (2) befindet, einen Kompressionsarm (30, 37) und eine Verriegelungsgabel (34) zum Aktivieren und Deaktivieren des Gewichtsaufnahmeelements (6) umfasst, wobei der Kompressionsarm (30, 37) über ein Kompressionsfedergehäuse (30.1), ein drittes Gehäuse des Kniegelenks (17.3) und eine Welle (24) mit dem Kniegelenk (4) und über ein Gewichtsaufnahmegehäuse (30.2) mit dem Gewichtsaufnahmeelement (6) verbunden ist,
wobei ein unteres Ende eines Stützarms (33) über Unterschenkelgehäuse (2.1) mit dem Unterschenkel (2) verbunden ist und das andere Ende des Stützarms (33) über ein Verriegelungsgabelgehäuse (34.1) mit der Verriegelungsgabel (34) verbunden ist.

2. Eine Prothesenvorrichtung (C) nach Anspruch 1, wobei das Gewichtsaufnahmeelement (6) eine Feder ist.

3. Eine Prothesenvorrichtung (C) nach Anspruch 1 oder 2, ferner umfassend einen Haltungsanpassungsmechanismus (DA), der eine Führungsschiene (79) und einen mit dem Kompressionsarm (30, 37) verbundenen Mitnehmer bzw. Träger (80) umfasst, wobei der Haltungsanpassungsmechanismus (DA) den Kompressionsarm (30, 37) in der Knieachse durch die Bewegung des Trägers (80) auf der Führungsschiene (79) in Übereinstimmung mit der durch das Potentiometer (19) durch Messung des Winkels und der Winkelgeschwindigkeit des Knies ermittelten Gehgeschwindigkeit einstellt, um das um das Knie erzeugte Drehmoment in Übereinstimmung mit der Gehgeschwindigkeit zu erhöhen oder zu verringern.

4. Eine Prothesenvorrichtung (C) nach einem der vorhergehenden Ansprüche, ferner umfassend einen Knöchelarretiermechanismus (K), der ein erstes Knöchelarretierelement (52), ein zweites Knöchelarretierelement (53), ein drittes Knöchelarretierelement (54), ein viertes Knöchelarretierelement (55) und ein fünftes Knöchelarretierelement (56) umfasst, wobei der Knöchelarretiermechanismus (K) ferner ein Knöchelwellenzahnrad (51) umfasst, das so konfiguriert ist, dass es das Knöchelgelenk durch Eingriff mit dem ersten Knöchelarretierelement (52) arretiert, falls das fünfte Knöchelarretierelement (56) den Boden berührt.

5. Eine Prothesenvorrichtung (C) nach einem der vorhergehenden Ansprüche, wobei der Gewichtsaufnahmemechanismus (3), der so konfiguriert ist, dass er eine Beugung (Flexion) des Knies von 75° bis 110° im Sitzen ermöglicht, so konfiguriert ist, dass er das Gewichtsaufnahmeelement (6) über den mit dem Kniegelenk (4) und dem Gewichtsaufnahmeelement (6) verbundenen Kompressionsarm (30, 37) beaufschlagt und Energie im Gewichtsaufnahmeelement (6) speichert.

6. Eine Prothesenvorrichtung (C) nach einem der vorhergehenden Ansprüche, wobei Fersenfedern (49), die so konfiguriert sind, dass sie eine Stoßdämpfung bereitstellen, am unteren Ende mittels eines rechten Fersenfedersitzes (46) und eines linken Fersenfedersitzes (47) mit dem Fußteil (5) verbunden sind und am oberen Ende mittels eines Fersenfedersverbinders (50) mit der rechten und linken Seite des Fußes (41, 42) verbunden sind.

7. Eine Prothesenvorrichtung (C) nach einem der vorhergehenden Ansprüche, ferner umfassend einen Motor (81), der an einem Ende des Gewichtsaufnahmeelements (6) angeordnet ist und durch Drücken des besagten Gewichtsaufnahmeelements (6) Leistung bereitstellt.

8. Eine Prothesenvorrichtung (C) nach einem der vorhergehenden Ansprüche, ferner umfassend eine Steuereinheit, die so angepasst ist, dass sie die in der Prothesen- oder Orthesenvorrichtung (C) enthaltenen Motoren (81) steuert.

## Revendications

1. Dispositif de prothèse (C) comprenant :
- une articulation de genou (4) pouvant être reliée à la jambe de l'utilisateur ;
- une partie inférieure de jambe (2) reliant l'articulation de genou (4) et une partie de pied (5) l'une à l'autre,
dans lequel la partie inférieure de jambe (2) est reliée à l'articulation de genou (4), et la partie inférieure de jambe (2) est reliée à la partie de pied (5) par l'intermédiaire d'un logement d'élément de cheville (41.1) ;
- un mécanisme de stockage d'oscillation (1) relié à la partie de pied (5) au moyen d'un logement de tube inférieur (16) et d'une goupille de tige à engrenage (68), et à l'articulation de genou (4) au moyen d'un logement de tube supérieur (7) et d'un premier logement d'articulation de genou (17.1),
- la partie de pied (5) comprend un ressort de dorsiflexion (60) et un mécanisme à ressort et engrenage de stockage d'oscillation (D), dans lequel le mécanisme à ressort et engrenage de stockage d'oscillation (D) comprend un ressort d'équilibrage (77) inséré dans le logement d'élément de cheville (41.1) situé dans un élément de cheville (44), dans lequel le ressort d'équilibrage (77) est configuré pour permettre au pied, ainsi qu'à la cheville, de revenir à sa position normale après la poussée, le mécanisme à ressort et engrenage de stockage d'oscillation (D) comprend en outre un engrenage moteur (63), un engrenage entraîné (72), un engrenage intermédiaire (76) et une tige à engrenage (70), dans lequel l'engrenage moteur (63) est configuré pour tourner par rapport au mouvement de la cheville, l'engrenage intermédiaire (76) est configuré pour transférer le mouvement de l'engrenage moteur (63) à l'engrenage entraîné (72) qui est configuré pour permettre à la tige à engrenage (70) et au mécanisme de stockage d'oscillation (1) fixé à ladite tige à engrenage (70) de se déplacer en va-et-vient, pour permettre au mécanisme de stockage d'oscillation (1) d'être transporté entre au moins deux positions dans la partie de pied (5) au moyen du mécanisme à ressort et engrenage de stockage d'oscillation (D) ;
dans lequel le mécanisme de stockage d'oscillation (1) comprend un mécanisme d'adaptation d'oscillation constitué de tubes d'élément de raccordement emboîtés (8, 15) et d'un ressort de stockage d'oscillation d'élément de raccordement (10) situé dans les tubes, un couvercle de ressort (9), une vis de guidage (13) et un moteur BLDC (14), et le mécanisme d'adaptation d'oscillation (1) est configuré pour modifier la position du ressort de stockage d'oscillation (10) et pour activer le ressort de stockage d'oscillation (10) à des moments différents en fonction de différentes vitesses de marche et ajuste l'angle d'oscillation du genou,
dans lequel un tube de ressort de dorsiflexion (57) contenant le ressort de dorsiflexion (60) est relié à l'avant de la partie de pied (5) par l'intermédiaire de logements de bout d'orteil (83), et une tige de piston (82) appuyant sur le tube de ressort de dorsiflexion (57) est reliée à l'élément de cheville (44) à l'arrière du pied,
le dispositif de prothèse (C) comprenant également :
- un mécanisme de réception de poids (3) comprenant un élément de réception de poids (6) situé dans la partie inférieure de jambe (2), un bras de compression (30, 37) et une fourche de verrouillage (34) pour activer et désactiver l'élément de réception de poids (6),
dans lequel le bras de compression (30, 37) est relié à l'articulation de genou (4) par l'intermédiaire d'un logement de ressort de compression (30.1) et d'un troisième logement d'articulation de genou (17.3) et d'un arbre (24), et à l'élément de réception de poids (6) par l'intermédiaire d'un logement de réception de poids (30.2),
dans lequel une extrémité inférieure d'un bras de support (33) est reliée à la partie inférieure de jambe (2) par l'intermédiaire de logements de partie inférieure de jambe (2.1) et l'autre extrémité du bras de support (33) est reliée à la fourche de verrouillage (34) par l'intermédiaire d'un logement de fourche de verrouillage (34.1).

2. Dispositif de prothèse (C) selon la revendication 1, dans lequel l'élément de réception de poids (6) est un ressort.

3. Dispositif de prothèse (C) selon la revendication 1 ou 2, comprenant en outre un mécanisme d'adaptation de posture (DA) comprenant un rail de guidage (79) et un chariot (80) relié au bras de compression (30, 37), dans lequel le mécanisme d'adaptation de posture (DA) ajuste le bras de compression (30, 37) dans l'axe du genou par le mouvement du chariot (80) sur le rail de guidage (79) en fonction de la vitesse de marche déterminée par le potentiomètre (19) en mesurant l'angle et la vitesse angulaire du genou, afin d'augmenter ou de diminuer le couple formé autour du genou en fonction de la vitesse de marche.

4. Dispositif de prothèse (C) selon l'une quelconque des revendications précédentes, comprenant en outre un mécanisme de verrouillage de cheville (K) comprenant un premier élément de verrouillage de cheville (52), un deuxième élément de verrouillage de cheville (53), un troisième élément de verrouillage de cheville (54), un quatrième élément de verrouillage de cheville (55) et un cinquième élément de verrouillage de cheville (56), le mécanisme de verrouillage de cheville (K) comprenant en outre un engrenage d'arbre de cheville (51) configuré pour verrouiller l'articulation de cheville en s'engageant avec le premier élément de verrouillage de cheville (52) dans le cas où le cinquième élément de verrouillage de cheville (56) touche le sol.

5. Dispositif de prothèse (C) selon l'une quelconque des revendications précédentes, dans lequel le mécanisme de réception de poids (3), qui est configuré pour permettre une flexion de 75° à 110° du genou en position assise, est configuré pour comprimer l'élément de réception de poids (6) via le bras de compression (30, 37) relié à l'articulation de genou (4) et à l'élément de réception de poids (6) et pour stocker de l'énergie sur l'élément de réception de poids (6).

6. Dispositif de prothèse (C) selon l'une quelconque des revendications précédentes, dans lequel des ressorts de talon (49) configurés pour assurer un amortissement des chocs sont reliés à la partie de pied (5) à l'extrémité inférieure au moyen d'un support de ressort de talon droit (46) et d'un support de ressort de talon gauche (47), et sont reliés aux côtés droit et gauche du pied (41, 42) à l'extrémité supérieure au moyen d'un raccord de ressort de talon (50).

7. Dispositif de prothèse (C) selon l'une quelconque des revendications précédentes, comprenant en outre un moteur (81) situé à une extrémité de l'élément de réception de poids (6) et fournissant de la puissance en comprimant ledit élément de réception de poids (6).

8. Dispositif de prothèse (C) selon l'une quelconque des revendications précédentes, comprenant en outre une unité de commande adaptée pour commander les moteurs (81) contenus dans le dispositif prothétique ou orthotique (C).
